# EUROPEAN PATENT APPLICATION

(11) **EP 1 514 537 A2**
(43) Date of publication of application: **16.03.2005**
(21) Application number: 04733645.8
(22) Date of filing: 18.05.2004
(51) Int. Cl.: A61K 7/48

(54) **ANTI-AGING AGENT AND COLLAGEN PRODUCTION PROMOTER**

(30) Priority: 11.03.2004 JP 2004068413
(71) Applicant: SHISEIDO COMPANY LIMITED, Chuo-ku, Tokyo 104-8010 (JP)
(72) Inventor: TAKADA, Keiko, SHISEIDO RESEARCH C.SHIN-YOKOHAMA, Yokohama-shi, Kanagawa 2248558 (JP); SUZUKI, Rikako, SHISEIDO RESEARCH C. SHIN-YOKOHAMA, Yokohama-shi, Kanagawa 2248558 (JP); GUENTERT, Miki, SHISEIDO RESEARCH C.SHIN-YOKOHAMA, Yokohama-shi, Kanagawa 2248558 (JP); INOMATA, Shinji, SHISEIDO RESEARCH C.SHIN-YOKOHAMA, Yokohama-shi, Kanagawa 2248558 (JP); HAMADA, Chika, SHISEIDO RESEARCH C.SHIN-YOKOHAMA, Yokohama-shi, Kanagawa 2248558 (JP); SAKIGUCHI, Takayuki, Yokohama-shi, Kanagawa 2248558 (JP)
(74) Representative: Becker Kurig Straus
(86) International application number: PCT/JP2004/007038
(87) International publication number: WO 2004/075621

(57) **Abstract**

There are provided an anti-aging composition, a collagen production promoting composition, a collagen gel contraction promoting composition, and an integrin production promoting composition for promoting integrin production in fibroblasts/epidermal cell, which have improved collagen production promotion effect and are effective for preventing or reducing wrinkle and/or sagging caused by aging. These anti-aging composition and promoting composition comprise, as an active ingredient, a hydrogen peroxide-treated yeast hydrolysate.

## Description

### TECHNICAL FIELD

The present invention relates to an anti-aging composition and a collagen production promoting composition and, more particularly, to an anti-aging composition and a collagen production promoting composition that can promote the production of collagen which is one of the components of the extracellular matrix. Further, the present invention relates to a promoting composition and a promoting method for collagen gel contraction, and to fibroblast or epidermal cell integrin production.

### BACKGROUND ART

Wrinkle and sagging observed in aged skin are a major change in appearance caused by aging and are a serious problem for many middle-aged and elderly persons. Thinning of skin tissues due to aging is one of the causes of wrinkle and sagging of the skin. In the aged skin, the amount of collagen fibers, which are a major matrix component of the dermis, is much lower than that in the non-aged skin. This is a major cause of the reduced skin thickness. Accordingly, keeping the amount of collagen on a suitable level by promoting collagen production is considered effective for preventing and reducing wrinkle and sagging of the skin.

For example, isoflavone compounds, selected from daidzein, daidzin, genistein, and genistin, and phytosterols have hitherto been reported as natural product-derived ingredients which can promote collagen production to prevent and reduce a change in skin caused by aging.

In recent years, however, there has been a strong demand for other substances having significant collagen production promotion effect.

### DISCLOSURE OF THE INVENTION

The present inventor has made extensive and intensive studies on substances having the effect of strongly promoting dermal fibroblast collagen biosynthesis. As a result, the present inventor has found that a hydrogen peroxide-treated yeast hydrolysate, which has been cultured in a specific nutrient medium, has significant collagen production promotion effect.

According to the present invention, there is provided an anti-aging composition characterized by comprising a collagen production promoting composition comprising, as an active ingredient, a hydrogen peroxide-treated yeast hydrolysate which has been cultured in a nutrient medium containing a non-animal-derived glycosaminoglycan. Further, there is provided a collagen production promoting composition characterized by comprising, as an active ingredient, a hydrogen peroxide-treated yeast hydrolysate which has been cultured in a nutrient medium containing a non-animal-derived glycosaminoglycan. Furthermore, there is provided a method for promoting collagen production, characterized by comprising using the above hydrogen peroxide-treated yeast hydrolysate to promote the production of collagen.

The hydrogen peroxide-treated yeast hydrolysate used in the present invention is known to have elastin production promotion effect. Up to now, however, the collagen production promotion effect of the hydrogen peroxide-treated yeast hydrolysate was not known and was found for the first time by the present inventor. In this connection, it should be noted that the elastin production promotion effect is a technical matter different from the collagen production promotion effect, and a substance having an elastin production promotion effect does not always have a collagen production promotion effect.

Further, regarding the hydrogen peroxide-treated yeast hydrolysate, the present inventor has made further studies on collagen gel contraction and epidermal cell integrin production promotion which are known to be involved in the prevention/amelioration of skin aging phenomena, for example, wrinkles and sagging. As a result, the present inventor has surprisingly found that this hydrolysate also has collagen gel contraction promotion effect, a fibroblast integrin production promotion effect, particularly for fibroblast integrin α2, integrin α3, integrin α6, integrin α2β1 and/or integrin β1 production promotion effect, as well as an epidermal cell integrin production promotion effect, particularly for epidermal cell integrin α2, integrin α3, integrin α6, integrin α2β1, integrin β1 and/or integrin β4 production promotion effect. Collagen gel contraction promotion as well as fibroblast and epidermal cell integrin production promotion are known to be effective for preventing skin aging and for ameliorating skin aging condition (for example, Japanese Unexamined Patent Publication (Kokai) Nos. 2001-39850, 10(1998)-72336, 2001-278769, and 2003-171225). Therefore, having such effects supports the anti-aging effect of the hydrogen peroxide-treated yeast hydrolysate used in the present invention.

Therefore, according to another aspect of the present invention, there is provided an anti-aging composition characterized by comprising, a collagen gel contraction promoting composition comprising, as an active ingredient, a hydrogen peroxide-treated yeast hydrolysate which has been cultured in a nutrient medium containing a non-animal-derived glycosaminoglycan. Further, there is provided a collagen gel contraction promoting composition characterized by comprising, as an active ingredient, a hydrogen peroxide-treated yeast hydrolysate which has been cultured in a nutrient medium containing a non-animal-derived glycosaminoglycan. Furthermore, there is provided a method for promoting collagen gel contraction, characterized by comprising using the above hydrogen peroxide-treated yeast hydrolysate to promote the contraction of a collagen gel.

According to a further aspect of the present invention, there is provided an anti-aging composition characterized by comprising an integrin production promoting composition for promoting integrin production by fibroblasts, said integrin production promoting composition comprising, as an active ingredient, a hydrogen peroxide-treated yeast hydrolysate which has been cultured in a nutrient medium containing a non-animal-derived glycosaminoglycan. Further, there is provided an integrin production promoting composition for promoting integrin production by fibroblasts, characterized in that said integrin production promoting composition comprises, as an active ingredient, a hydrogen peroxide-treated yeast hydrolysate which has been cultured in a nutrient medium containing a non-animal-derived glycosaminoglycan. Furthermore, there is provided a method for promoting fibroblast integrin production, characterized by comprising using the above hydrogen peroxide-treated yeast hydrolysate to promote fibroblast integrin production.

According to a still further aspect of the present invention, there is provided an anti-aging composition characterized by comprising an integrin production promoting composition for promoting epidermal cell integrin production, said integrin production promoting composition comprising, as an active ingredient, a hydrogen peroxide-treated yeast hydrolysate which has been cultured in a nutrient medium containing a non-animal-derived glycosaminoglycan. Further, there is provided an integrin production promoting composition for promoting epidermal cell integrin production, characterized by comprising, as an active ingredient, a hydrogen peroxide-treated yeast hydrolysate which has been cultured in a nutrient medium containing a non-animal-derived glycosaminoglycan. Furthermore, there is provided a method for promoting epidermal cell integrin production, characterized by comprising using the above hydrogen peroxide-treated yeast hydrolysate to promote epidermal cell integrin production.

### BEST MODE FOR CARRYING OUT THE INVENTION

The present invention will be described in detail.

The hydrogen peroxide-treated yeast hydrolysate used in the present invention is a water-soluble yeast hydrolysate produced by treating yeast in aqueous hydrogen peroxide, preferably a water-soluble yeast hydrolysate produced by subjecting yeast to the above treatment and further exposing the treated product to ultraviolet radiation. Details of the hydrogen peroxide-treated yeast hydrolysate including the production process thereof are described in U.S. Patent No. 6461857. Further, the hydrogen peroxide-treated yeast hydrolysate used in the present invention is commercially available from Arch Personal Care Products, L.P. under the tradename designation "Biodynes EMPP (trademark)."

The preparation of the hydrogen peroxide-treated yeast hydrolysate used in the present invention is based on a mechanism where, when culture yeast is cultured under stress applied by hydrogen peroxide, the yeast responds to the stress to produce a cell protective component which protects cells from the stress. Specifically, the hydrogen peroxide-treated yeast hydrolysate can be prepared by adding hydrogen peroxide to cultured yeast cells and culturing the mixture in a nutrient medium containing a non-animal-derived glycosaminoglycan.

More specifically, for example, the hydrogen peroxide-treated yeast hydrolysate according to the present invention can be prepared according to the following procedure:
(a) provide a culture of yeast cells, for-example, Saccharomyces cerevisae;
(b) add a sub-lethal amount, for example, about 0.1 to 2% by mass based on the total mass of the culture, of hydrogen peroxide to the culture to apply stress to the cells;
(C) optionally expose the culture to a sub-lethal amount of ultraviolet radiation (for example, UVA/UVB irradiation at an intensity of 31.5 mJ/cm²) to apply stress to the cells; and
(d) obtain a water-soluble yeast extract from the hydrogen peroxide-containing culture.

The anti-aging composition, collagen production promoting composition, collagen gel contraction composition, fibroblast integrin production promoting composition, and epidermal cell integrin production promoting composition according to the present invention can be formulated to contain, in addition to the above indispensable ingredient, optional ingredients commonly used in external preparations for skin such as cosmetics and pharmaceutical products, for example, skin-whitening agents, moisturizing agents, antioxidants, oil components, ultraviolet absorbers, surfactants, thickeners, alcohols, powder components, coloring materials, aqueous components, water, and various nutrient preparations for skin.

Further, other materials, for example, disodium edetate, trisodium edetate, sodium citrate, sodium polyphosphate, sodium metaphosphate, gluconic acid, and other sequestering agents, caffeine, tannin, verapamil, tranexamic acid and its derivatives, licorice extract, glabridin, hot-water extracts of fruits of Kakyoku (Pyracantha fortuneana), various crude drugs, tocopherol acetate, glycyrrhetinic acid and its derivatives or its salts, and other drugs, vitamin C, magnesium ascorbic acid phosphate, ascorbic acid glucoside, arbutin, kojic acid, and other skin-whitening agents, glucose, fructose, mannose, sucrose, trehalose, and other saccharides may also be optionally formulated.

The present invention can be extensively applied to cosmetics, quasidrugs and the like used for the outer skin, particularly preferably to cosmetics. The compositions and the promoting compositions according to the present invention may take a wide variety of forms such as aqueous solutions, dissolvable forms, emulsions, powders, oils, gels, ointments, aerosols, water-oil two-phase systems, water-oil-powder three-phase systems and the like. That is, in the case of basic cosmetics, they can be extensively applied in the above various forms for face cleansers, skin lotions, milky lotions, creams, gels, essences (beauty care lotions), packs, masks and the like. Further, in the case of makeup cosmetics, they can be applied in a wide range of forms such as foundations, and, in the case of toiletry products, they can be applied in a wide range of forms such as body soaps, soaps and the like. Further, in the case of quasidrugs, they can be applied in a wide range of forms such as various ointments. It should be noted that the form and type of the collagen production promoter according to the present invention are not limited to these forms and types.

### EXAMPLES

The following Examples further illustrate but do not limit the present invention. Formulating amounts are in % by weight. Before the Examples, the methods for testing the effects and the results thereof will be explained.

### 1. Test Example 1 (Type I collagen assay)

### 1-1. Assay method

### (1) Culture of human fibroblasts

Human fibroblasts were inoculated in an amount of 1 x 10⁴ cells/well in a 10% fetal bovine serum-containing DMEM medium in a 96-well plate, and, 3 to 4 hrs after the inoculation, the concentration of the serum was decreased to 0.5%. One day after that, the medium was replaced with a 0.5% serum-containing DMEM medium with Biodynes EMPP being added thereto in varied concentrations. On the 4th day from the cell inoculation, the amount of type I collagen in the supernatant of the medium was measured, and the amount of DNA was measured for the cells as a measure of the number of cells.

### (2) Quantitative determination of DNA

The amount of DNA was measured by fluorimetry with H33342 manufactured by Hoechst.

### (3) Measurement of type I collagen

Procollagen type I carboxyterminal propeptide (PIP) produced by human fibroblasts was measured by ELISA with Kit MK-101 manufactured by Takara Shuzo Co., Ltd. The percentage of the amount of PIP per DNA of the sample relative to the amount of PIP per DNA in the control sample to be 100 was regarded as type I collagen production promotion rate.

For comparison, the collagen production promotion effect was examined in the same manner as described just above, except that "Biodynes TRF-25" (tradename: manufactured by Arch Personal Care Products L.P.), which is also a yeast culture hydrolysate, was used instead of "Biodynes EMPP" according to the present invention.

The production process of "Biodynes TRF-25" is different from the production process of "Biodynes EMPP" according to the present invention in that growth peptones with various low-molecular weights and non-animal-derived glycosaminoglycan were not provided in a satisfactory amount and, in addition, hydrogen peroxide treatment was not carried out.

### 1-2. Results

### (1) Results of quantitative determination of DNA

| | |
|---|---|
| Group without addition of Biodynes EMPP (control group) | 100% |
| Present composition 0.01% | 107% |
| Present composition 0.1% | 115% |

### (2) Results of measurement of type I collagen

| | |
|---|---|
| Group without addition of Biodynes EMPP (control group) | 100% |
| Present composition 0.001% | 136% |
| Present composition 0.01% | 172% |
| Present composition 0.1% | 186% |
| Present composition 1% | 232% |
| Biodynes TRF-25 0.001% | 106% |

### 2. Test example 2 (Type VII collagen assay)

### 2-1. Assay method

### (1) Culture of human fibroblasts

Human skin fibroblasts were inoculated in an amount of 1 x 10⁵ cells/well in a DMEM medium containing a 0.25% fetal bovine serum and 250 µM ascorbic acid glucoside in a 24-well plate. After cell adhesion, Biodynes EMPP was added. TWO days after, the medium was centrifuged. The supernatant thus obtained was measured for type VII collagen and the amount of DNA for cells as a measure of the number of cells.

### (2) Quantitative determination of DNA

The amount of DNA was measured by fluorimetry with H33342 manufactured by Hoechst.

### (3) Determination of type VII collagen by sandwich ELISA

Type VII collagen was measured by sandwich ELISA. In this Example, the following antibody was used.
· Type VII collagen-specific antibody
· Monoclonal antibody NP-185 and monoclonal antibody NP-32

The amount of type VII collagen per DNA of the agent added sample by setting the amount of type VII collagen per DNA in the sample, to which Biodynes EMPP had not been added, (control sample) to be 100 was regarded as type VII collagen production promotion rate.

### 2-2. Results

Results of measurement of type VII collagen

| | |
|---|---|
| Group without addition of Biodynes EMPP (control group) | 100% |
| Present composition 0.01% | 105% |
| Present composition 0.1% | 110% |
| Present composition 1% | 175% |
| Biodynes TRF-25 0.1% | 95% |
| Biodynes TRF-25 1% | 100% |

As is apparent from the above results, the group to which Biodynes EMPP had been added was much superior in type I collagen production promotion effect as compared with the group to which Biodynes TRF-25 had been added. Further, it has become apparent that the group to which Biodynes EMPP had been added also had type VII collagen production promotion effect.

It is considered that, due to the difference in production process between Biodynes EMPP and Biodynes TRF-25 (various stimulation methods for yeast), in the case of Biodynes EMPP, more useful cell activation components were produced and this caused the difference in the effect on fibroblasts between Biodynes EMPP and Biodynes TRF-25.

For the yeast extract obtained by extracting yeast with an aqueous medium, no collagen production promotion effect was observed. This result is also described in Japanese Patent No. 3278138.

### 3. Test Example 3 (Collagen gel contraction promotion ability assay)

### 3-1. Assay method

A human dermal fibroblast suspension collagen solution (using, as collagen, I-AC manufactured by KOKEN CO., LTD.) was prepared on ice and was mixed with a 0.25% FBS/DMEM medium containing Biodynes EMPP (purified water in the case of control; the concentration is expressed in mass%), and the mixture was then poured into a 3.5-cm dish. After gelation at 37°C, the gel was separated from the wall surface of the dish so as to allow collagen gel contraction. One to four days after, the diameter of the collagen gel was measured from three directions, and the measured values were averaged. The percentage contraction after the addition of the test substance was determined by setting the diameter before the contraction to be contraction 0% (for each group, n = 3 or 4).

### 3-2. Results

Results of collagen gel contraction promotion ability assay

| | | |
|---|---|---|
| Group without addition of Biodynes EMPP (control group) | After one day | 11% |
| | After two days | 20% |
| | After three days | 26% |
| | After four days | 28% |
| Present composition 0.03% | After one day | 17% |
| | After two days | 28% |
| | After three days | 35% |
| | After four days | 39% |
| Present composition 0.1% | After one day | 20% |
| | After two days | 29% |
| | After three days | 37% |
| | After four days | 42% |
| Present composition 0.3% | After one day | 24% |
| | After two days | 36% |
| | After three days | 46% |
| | After four days | 50% |

As is apparent from the above results, the group to which Biodynes EMPP had been added was much superior in collagen gel contraction promotion effect to the control group, and it was also confirmed that the collagen gel contraction promotion effect depends upon the concentration of Biodynes EMPP.

### 4. Test Example 4 (Assay on integrin production promotion ability in dermal fibroblasts)

### 4-1. Assay method

Biodynes EMPP was allowed to act on human dermal fibroblasts, and, 24 hr after the action, the cells were separated by trypsin/EDTA. After neutralization with FCS, the cells were washed with PBS containing 0.01% FCS and 0.02% NaN₃ to recover the cells. Anti-human integrin α2, anti-human integrin α3, anti-human integrin α6, anti-human integrin α2β1, and anti-human integrin β1 were each used after 100-fold dilution as a primary antibody, and FITC-labeled anti-mouse IgG1 was used after 100-fold dilution as a secondary antibody. In the control, mouse IgGl was used as the primary antibody. The amount of integrin on the cell surface was measured with FACScan.

### 4-2. Results

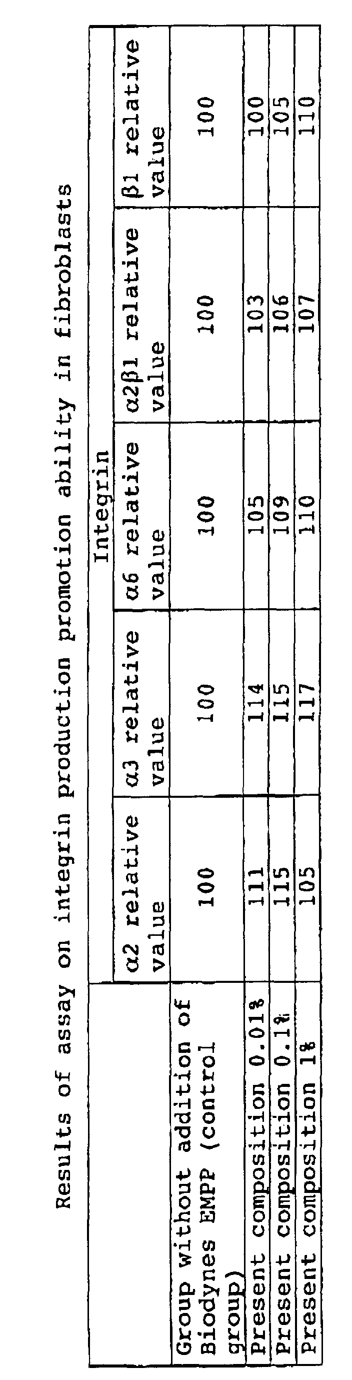

### 5. Test Example 5 (Assay on integrin production promotion ability in epidermal cells)

### 5-1. Assay method

Biodynes EMPP was allowed to act on epidermal cells (HaCaT cells), and the amount of integrin on the surface of cells was measured with FACScan in the same manner as in Test Example 4. Anti-human integrin α2, anti-human integrin α3, anti-human integrin α6, anti-human integrin α2β1, anti-human integrin β1, and anti-human integrin β4 were each used after 100-fold dilution as a primary antibody.

### 5-2. Results

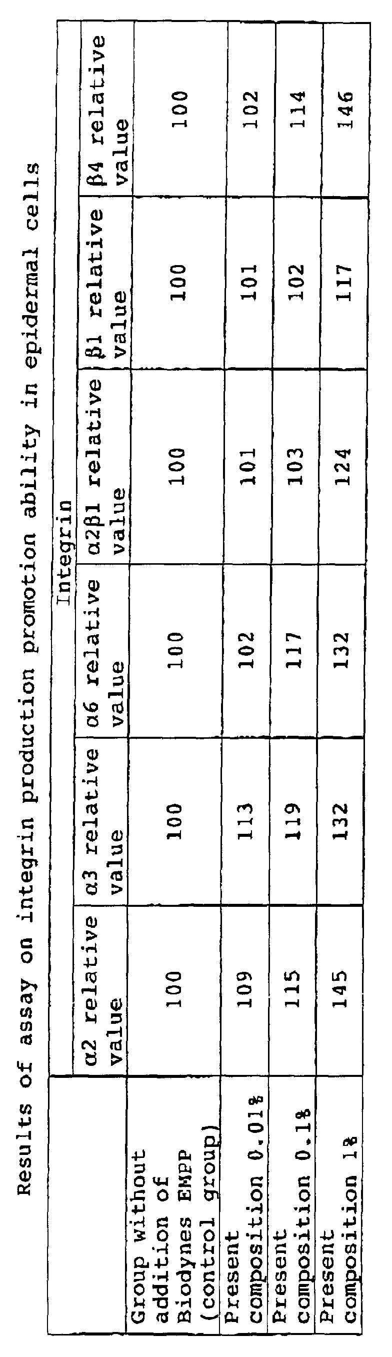

As is apparent from the results, the group to which Biodynes EMPP had been added could enhance integrin production in both fibroblasts and epidermal cells.

| [Example 1] O/W cream | |
|---|---|
| Liquid paraffin | 3.0 mass% |
| Vaseline | 1.0 |
| Dimetyl polysiloxane | 1.0 |
| Stearyl alcohol | 1.8 |
| Behenyl alcohol | 1.6 |
| Macadamia nut oil | 2.0 |
| Hydrogenated palm oil | 3.0 |
| Squalane | 6.0 |
| Stearic acid | 2.0 |
| Cholesteryl hydroxystearate | 0.5 |
| Cetyl 2-ethylhexanoate | 4.0 |
| Polyoxyethylene hydrogenated castor oil | 0.5 |
| Self-emulsifiable glycerin monostearate | 3.0 |
| Tocopherol acetate | 0.1 |
| Retinol | 0.01 |
| (Dimethicone/vinyldimethicone) crosspolymer (tradename: KSG-16, The Shin-Etsu Chemical Co., Ltd.) | 3.0 |
| 4-t-Butyl-4'-methoxydibenzoylmethane | 0.05 |
| p-Oxybenzoic ester | q.s. |
| Mono-2-ethylhexanoic acid di-p-methoxycinnamic acid glyceryl ester | 0.05 |
| Potassium hydroxide | 0.15 |
| Sodium hexametaphosphate | 0.05 |
| Trimethylglycine | 2.0 |
| Glycerin | 8.0 |
| Dipropylene glycol | 5.0 |
| Potassium salt of α-tocopherol 2-L-ascorbic acid phosphoric acid diester | 1.0 |
| (Ammonium acryloyldimethyltaurate/VP) copolymer (tradename: Aristoflex AVC, Clariant) | 0.1 |
| Rubussuavissimus extract | 0.1 |
| Biodynes EMPP | 0.1 |
| Trisodium edetate | 0.05 |
| Carboxyvinyl polymer | 0.05 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 10.0 |
| Mica titanium | 0.1 |
| Coloring material | q.s. |
| Purified water | Balance |

| [Example 2] W/O cream for tautness | |
|---|---|
| Dimethyl polysiloxane | 3.0 mass% |
| Decamethylcyclopentasiloxane | 13.0 |
| Dodecamethylcyclohexasiloxane | 5.0 |
| Polyoxyethylene/methylpolysiloxane copolymer | 1.0 |
| 3-Tris(trimethylsiloxy)silylpropylcarbamide acid pullulan | 1.0 |
| Dow Corning 9040 silicone elastomer blend (blend of dimethicone crosspolymer with decamethylcyclopentasiloxane, Dow Corning) | 5.0 |
| Retinol acetate | 0.1 |
| p-Oxybenzoic ester | q.s. |
| L-Menthol | q.s. |
| Trimethylsiloxysilicic acid | 2.0 |
| Ethanol | 2.0 |
| Glycerin | 3.0 |
| Dipropylene glycol | 5.0 |
| Polyethylene glycol 6000 | 5.0 |
| Sodium hexametaphosphate | 0.05 |
| Tocopherol acetate | 0.1 |
| Caffeine | 0.1 |
| Fennel extract | 0.1 |
| Hamamelis extract | 0.1 |
| Ginseng extract | 0.1 |
| Biodynes EMPP | 1.0 |
| Trisodium edetate | 0.05 |
| Dimorpholinopyridazinone | 0.01 |
| Methylbis(trimethylsiloxy)silylisopentyl trimethoxycinnamate | 0.1 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 5.0 |
| Yellow iron oxide | q.s. |
| Cobalt titanate | q.s. |
| Dimethyl distearyl ammonium hectorite | 1.5 |
| Polyvinyl alcohol | 0.1 |
| Hydroxyethyl cellulose | 0.1 |
| Sodium acrylate/2-acrylamide-2-methylpropane sulfonic acid copolymer (tradename: SIMULGEL EG, SEPPIC) | 0.1 |
| (Ammonium acryloyldimethyltaurate/VP) copolymer (tradename: Aristoflex AVC, Clariant) | 0.1 |
| Perfume | q.s. |
| Purified water | Balance |

| [Example 3] O/W cream for wrinkles | |
|---|---|
| Liquid paraffin | 8.0 mass% |
| Vaseline | 3.0 |
| Dimetyl polysiloxane | 2.0 |
| Dow Corning 9041 silicone elastomer blend (blend of dimethicone crosspolymer with dimethylpolysiloxane, Dow Corning) | 0.1 |
| Stearyl alcohol | 3.0 |
| Behenyl alcohol | 2.0 |
| Glycerin | 5.0 |
| Dipropylene glycol | 4.0 |
| Trehalose | 1.0 |
| Pentaerythrit tetra-2-ethylhexanoate | 4.0 |
| Polyoxyethylene glyceryl monoisostearate | 2.0 |
| Polyoxyethylene glyceryl monostearate | 1.0 |
| Lipophilic glyceryl monostearate | 2.0 |
| Citric acid | 0.05 |
| Sodium citrate | 0.05 |
| Potassium hydroxide | 0.015 |
| Oil-soluble licorice extract | 0.1 |
| Sprout extract of Fagus crenata | 0.5 |
| Retinol palmitate (1,000,000 units) | 0.25 |
| Tocopherol acetate | 0.1 |
| Biodynes EMPP | 5.0 |
| p-Oxybenzoic ester | q.s. |
| Phenoxy ethanol | q.s. |
| Dibutylhydroxytoluene | q.s. |
| Trisodium edetate | 0.05 |
| 4-t-Butyl-4'-methoxydibenzoylmethane | 0.01 |
| 2-Ethylhexyl p-methoxycinnamate | 0.1 |
| β-Carotene | 0.01 |
| Polyvinyl alcohol | 0.5 |
| Hydroxyethyl cellulose | 0.5 |
| Carboxyvinyl polymer | 0.05 |
| (Ammonium acryloyldimethyltaurate/VP) copolymer (tradename: Aristoflex AVC, Clariant) | 5.0 |
| Purified water | Balance |
| Perfume | q.s. |

| [Example 4] O/W cream for moisture retention | |
|---|---|
| Liquid paraffin | 10.0 mass% |
| Dimetyl polysiloxane | 2.0 |
| Dow Corning 9045 silicone elastomer blend (blend of dimethicone crosspolymer with decamethylcyclopentasiloxane, Dow Corning) | 3.0 |
| Glycerin | 10.0 |
| 1,3-Butylene glycol | 2.0 |
| Erythritol | 1.0 |
| Polyethylene glycol 1500 | 5.0 |
| Squalane | 15.0 |
| Pentaerythrit tetra-2-ethylhexanoate | 5.0 |
| Potassium hydroxide | 0.1 |
| Sodium hexametaphosphate | 0.05 |
| Tocopherol acetate | 0.05 |
| Biodynes EMPP | 0.1 |
| Peach kernel extract | 1.0 |
| p-Oxybenzoic ester | q.s. |
| Hydroxypropylmethylcellulose | 0.3 |
| Polyvinyl alcohol | 0.1 |
| Carboxyvinyl polymer | 0.2 |
| Sodium acrylate/2-acrylamide-2-methylpropane sulfonic acid copolymer (tradename: SIMULGEL EG, SEPPIC) | 0.5 |
| Acrylate/alkyl methacrylate copolymer (PEMULEN TR-2) | 0.1 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 5.0 |
| Purified water | Balance |

| [Example 5] W/O sunscreen | |
|---|---|
| Dimethylpolysiloxane | 5.0 mass% |
| Decamethylcyclopentasiloxane | 10.0 |
| Trimethylsiloxysilicic acid | 3.0 |
| 3-Tris(trimethylsiloxy)silylpropylcarbamide acid pullulan | 5.0 |
| Polyoxyethylene/methyl polysiloxane copolymer | 3.0 |
| (Dimethicone/vinyldimethicone) crosspolymer (tradename: KSG-16, The Shin-Etsu Chemical Co., Ltd.) | 30.0 |
| Dipropylene glycol | 3.0 |
| Cetyl 2-ethylhexanoate | 1.0 |
| Silicone-coated zinc oxide fine particles (60 nm) | 10.0 |
| Talc | 1.0 |
| Silicone-coated titanium oxide fine particles (40 nm) | 7.0 |
| Biodynes EMPP | 1.0 |
| Hypotaurine | 0.1 |
| Sophora angustifolia extract | 0.01 |
| Adenosine triphosphate disodium | 0.5 |
| Wild rose extract | 1.0 |
| p-Oxybenzoic ester | q.s. |
| Phenoxy ethanol | q.s. |
| Trisodium edetate | 0.2 |
| Dimethyl distearyl ammonium hectorite | 1.0 |
| Spherical powder of polymethyl methacrylate copolymer | 3.0 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Mica titanium | 0.5 |
| Purified water | Balance |
| Perfume | q.s. |

| [Example 6] O/W cream | |
|---|---|
| Glycerin | 3.0 mass% |
| Decamethylcyclopentasiloxane | 7.0 |
| Dimethylpolysiloxane | 5.0 |
| Polyoxyethylenesorbitan monooleate (20 E.O.) | 1.0 |
| Polyethylene glycol 20000 | 0.5 |
| Polyvinyl alcohol | 1-0 |
| Ethanol | 5.0 |
| 3-Tris(trimethylsiloxy)silylpropylcarbamide acid pullulan | 3.0 |
| Trimethylsiloxysilicic acid | 0.1 |
| Dimorpholinopyridazinone | 0.1 |
| Potassium hydroxide | 0.12 |
| Mica titanium | 0.1 |
| Dow Corning 9045 silicone elastomer blend (blend of dimethicone crosspolymer with decamethylcyclopentasiloxane, Dow Corning) | 20.0 |
| (Ammonium acryloyldimethyltaurate/VP) copolymer (tradename: Aristoflex AVC, Clariant) | 2.0 |
| Sodium acrylate/2-acrylamide-2-methylpropane sulfonic acid copolymer (tradename: SIMULGEL EG, SEPPIC) | 0.1 |
| Carbomer | 0.01 |
| Acrylate/alkyl methacrylate copolymer (PEMULEN TR-2) | 0.05 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 5.0 |
| Aluminum oxide | 0.01 |
| Xanthan gum | 0.1 |
| Sodium carboxymethylcellulose | 0.05 |
| Biodynes EMPP | 5.0 |
| Tranexamic acid | 1.0 |
| Retinol palmitate (1,000,000 units) | 0.01 |
| p-Oxybenzoic ester | q.s. |
| Phenoxy ethanol | q.s. |
| Perfume | q.s. |
| Ion-exchanged water | Balance |

| [Example 7] W/O cream | |
|---|---|
| Dipropylene glycol | 1.0 mass% |
| Decamethylcyclopentasiloxane | 5.0 |
| Dimethylpolysiloxane | 1.0 |
| Polyoxyethylene/methyl polysiloxane copolymer | 2.0 |
| Tri-2-ethylhexanoic acid glyceryl ester | 0.1 |
| Polyoxyethylenesorbitan monostearate (20 E.O.) | 0.05 |
| Polyethylene glycol 9000 | 2.0 |
| Polyvinyl alcohol | 0.5 |
| 3-Tris(trimethylsiloxy)silylpropylcarbamide acid pullulan | 5.0 |
| Dimorpholinopyridazinone | 0.1 |
| Potassium hydroxide | 0.03 |
| Aluminum oxide | 0.1 |
| Titanium oxide | 1.0 |
| Dow Corning 9041 silicone elastomer blend (blend of dimethicone crosspolymer with dimethylpolysiloxane, Dow Corning) | 40.0 |
| (Ammonium acryloyldimethyltaurate/VP) copolymer (tradename: Aristoflex AVC, Clariant) | 0.1 |
| Sodium acrylate/2-acrylamide-2-methylpropane sulfonic acid copolymer (tradename: SIMULGEL EG, SEPPIC) | 0.1 |
| Carbomer | 0.01 |
| Acrylate/alkyl methacrylate copolymer (PEMULEN TR-2) | 0.05 |
| Talc | 1.0 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Flaky inorganic composite powder (sericite which has been coated with titanium oxide, aluminum oxide, and silicic anhydride, tradename: COVERLEAF AR-20121 M, Catalysts and Chemicals Industries Co., Ltd.) | 1.0 |
| Spherical nylon powder | 1.0 |
| Silicic anhydride | 1.0 |
| Hydroxyethylcellulose | 0.1 |
| Biodynes EMPP | 1.0 |
| Soybean-fermented extract | 1.0 |
| p-Oxybenzoic ester | q.s. |
| Phenoxy ethanol | q.s. |
| Perfume | q.s. |
| Ion-exchanged water | Balance |

| [Example 8] O/W cream for prevention of wrinkles/sagging | |
|---|---|
| Glycerin | 5.0 mass% |
| Decamethylcyclopentasiloxane | 1.0 |
| Dodecamethylcyclohexasiloxane | 3.0 |
| Dimethylpolysiloxane | 5.0 |
| Methylphenylpolysiloxane | 1.0 |
| Octylmethoxycinnamate | 0.1 |
| Pentaerythrit tetra-2-ethylhexanoate | 1.0 |
| Polyoxyethylenesorbitan monolaurate (20 E.O.) | 0.1 |
| Polyoxyethylene/methyl polysiloxane copolymer (tradename: polyether-modified silicone oil, KF-6018, The Shin-Etsu Chemical Co., Ltd.) | 1.0 |
| Soybean-fermented extract | 3.0 |
| Polyethylene glycol 6000 | 2.0 |
| Citric acid | 0.1 |
| Sodium citrate | 0.1 |
| Sodium metaphosphate | 0.05 |
| 3-Tris(trimethylsiloxy)silylpropylcarbamide acid pullulan | 0.1 |
| (Dimethicone/vinyldimethicone) crosspolymer (tradename: KSG-16, The Shin-Etsu Chemical Co., Ltd.) | 10.0 |
| Dow Corning 9041 silicone elastomer blend (blend of dimethicone crosspolymer with dimethylpolysiloxane, Dow Corning) | 5.0 |
| (Ammonium acryloyldimethyltaurate/VP) copolymer (tradename: Aristoflex AVC, Clariant) | 0.1 |
| Ammonium acryloyldimethyltaurate/ Beheneth-25 methacrylate crosspolymer (tradename: Aristoflex HMB, Clariant) | 0.05 |
| Acrylate/alkyl methacrylate copolymer (PEMULEN TR-2) | 0.05 |
| Acrylamide/2-acrylamide-2-methylpropane sulfonic acid copolymer (tradename: SIMULGEL 600, SEPPIC) | 0.5 |
| Ammonium polyacrylate (tradename: SIMULGEL A, SEPPIC) | 0.5 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Flaky inorganic composite powder (sericite which has been coated with titanium oxide, aluminum oxide, and silicic anhydride, tradename: COVERLEAF AR-20121 M, Catalysts and Chemicals Industries Co., Ltd.) | 1.0 |
| (Dimethicone/vinyldimethicone) crosspolymer (tradename: BY29-119, Dow Corning Toray Silicone Co., Ltd.) | 1.0 |
| Biodynes EMPP | 1.0 |
| p-Oxybenzoic ester | q.s. |
| Phenoxy ethanol | q.s. |
| Perfume | q.s. |
| Ion-exchanged water | Balance |

| [Example 9] O/w milky lotion | |
|---|---|
| Dimethylpolysiloxane | 3.0 mass% |
| Decamethylcyclopentasiloxane | 4.0 |
| 3-Tris(trimethylsiloxy)silylpropylcarbamide acid pullulan | 0.5 |
| Dow Corning 9040 silicone elastomer blend (blend of dimethicone crosspolymer with decamethylcyclopentasiloxane, Dow Corning) | 0.5 |
| Ethanol | 5.0 |
| Glycerin | 6.0 |
| 1,3-Butylene glycol | 5.0 |
| Polyoxyethylene methyl glucoside | 3.0 |
| Sunflower oil | 1.0 |
| Squalane | 2.0 |
| Potassium hydroxide | 0.1 |
| Sodium hexametaphosphate | 0.05 |
| Hydroxypropyl-β-cyclodextrin | 0.1 |
| Dipotassium glycyrrhizinate | 0.05 |
| Loquat leaf extract | 0.1 |
| Sodium L-glutamate | 0.05 |
| Fennel extract | 0.1 |
| Lavender oil | 0.1 |
| Vitamin E | 0.1 |
| Extract of Rehmannia glutinosa Libosch | 0.1 |
| Hypotaurine | 0.01 |
| Thiotaurine | 0.1 |
| Mortierella oil | 0.5 |
| Arbutin | 3.0 |
| Sodium acetylhyaluronate | 0.1 |
| Marjoram extract | 0.1 |
| Biodynes EMPP | 0.1 |
| Dimorpholinopyridazinone | 0.1 |
| Xanthan gum | 0.1 |
| carboxyvinyl polymer | 0.1 |
| Acrylate/alkyl methacrylate copolymer (PEMULEN TR-1) | 0.1 |
| (Ammonium acryloyldimethyltaurate/VP) copolymer (tradename: Aristoflex AVC, Clariant) | 1.0 |
| Red iron oxide | q.s. |
| Yellow iron oxide | q.s. |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Mica titanium | 0.1 |
| p-Oxybenzoic ester | q.s. |
| Purified water | Balance |

| [Example 10] O/W milky lotion | |
|---|---|
| Dimethylpolysiloxane | 2.0 mass% |
| (Dimethicone/vinyldimethicone) crosspolymer (tradename: KSG-16, The Shin-Etsu Chemical Co., Ltd.) | 15.0 |
| Behenyl alcohol | 1.0 |
| Batyl alcohol | 0.5 |
| Glycerin | 5.0 |
| 1,3-Butylene glycol | 7.0 |
| Erythritol | 2.0 |
| Biodynes EMPP | 5.0 |
| Silica-coated zinc oxide | 0.1 |
| Pyrola japonica Klenze extract | 0.1 |
| Hydrogenated palm oil | 3.0 |
| Squalane | 6.0 |
| Pentaerythrit tetra-2-ethylhexanoate | 2.0 |
| Polyoxyethylene glyceryl isostearate | 1.0 |
| Polyoxyethylene glyceryl monostearate | 1.0 |
| Potassium hydroxide | q.s. |
| Sodium hexametaphosphate | 0.05 |
| Phenoxy ethanol | q.s. |
| Carboxyvinyl polymer | 0.1 |
| Sodium acrylate/2-acrylamide-2-methylpropane sulfonic acid copolymer (tradename: SIMULGEL EG, SEPPIC) | 0.1 |
| Purified water | Balance |

| [Example 11] O/W milky lotion | |
|---|---|
| Liquid paraffin | 7.0 mass% |
| Vaseline | 3.0 |
| Decamethylcyclopentasiloxane | 2.0 |
| 3-Tris(trimethylsiloxy)silylpropylcarbamide acid pullulan | 0.1 |
| Dow Corning 9041 silicone elastomer blend (blend of dimethicone crosspolymer with dimethylpolysiloxane, Dow Corning) | 3.0 |
| Behenyl alcohol | 0.5 |
| Glycerin | 5.0 |
| Dipropylene glycol | 7.0 |
| Polyethylene glycol 1500 | 2.0 |
| Jojoba oil | 1.0 |
| Isostearic acid | 0.5 |
| Stearic acid | 0.5 |
| Behenic acid | 0.5 |
| Pentaerythrit tetra-2-ethylhexanoate | 3.0 |
| Cetyl 2-ethylhexanoate | 3.0 |
| Glycerin monostearate | 1.0 |
| Polyoxyethylene glyceryl monostearate | 1.0 |
| Potassium hydroxide | 0.1 |
| Sodium hexametaphosphate | 0.05 |
| Stearyl glycyrrhetinate | 0.05 |
| L-Arginine | 0.1 |
| Trimethylglycine | 0.1 |
| Ascorbyl-2-glucoside | 0.1 |
| Xylitol | 1.0 |
| Royal jelly extract | 0.1 |
| Tocopherol acetate | 0.1 |
| Acetylated sodium hyaluronate | 0.1 |
| Biodynes EMPP | 1.0 |
| Trisodium edetate | 0.05 |
| 4-t-Butyl-4'-methoxydibenzoylmethane | 0.1 |
| 2-Ethylhexyl p-methoxycinnamate | 0.1 |
| Carboxyvinyl polymer | 0.15 |
| (Ammonium acryloyldimethyltaurate/VP) copolymer (tradename: Aristoflex AVC, Clariant) | 0.5 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 0.5 |
| Mica titanium | 0.1 |
| p-Oxybenzoic ester | q.s. |
| Purified water | Balance |
| Perfume | q.s. |

| [Example 12] W/O milky lotion for daytime | |
|---|---|
| Dimethyl polysiloxane | 2.0 mass% |
| Decamethylcyclopentasiloxane | 25.0 |
| Dodecamethylcyclohexasiloxane | 10.0 |
| Polyoxyethylene/methylpolysiloxane copolymer | 1.5 |
| 3-Tris(trimethylsiloxy)silylpropylcarbamide acid pullulan | 10.0 |
| Dow Corning 9045 silicone elastomer blend (blend of dimethicone crosspolymer with decamethylcyclopentasiloxane, Dow Corning) | 5.0 |
| Trimethylsiloxysilicic acid | 1.0 |
| 1,3-Butylene glycol | 5.0 |
| Squalane | 0.5 |
| Talc | 5.0 |
| Dipotassium glycyrrhizinate | 0.1 |
| Tocopherol acetate | 0.1 |
| Biodynes EMPP | 0.1 |
| L-Serine | 1.0 |
| Akebia extract | 0.1 |
| Trisodium edetate | 0.05 |
| 4-t-Butyl-4'-methoxydibenzoylmethane | 1.0 |
| 2-Ethylhexyl p-methoxycinnamate | 5.0 |
| Mono-2-ethylhexanoic acid di-p-methoxycinnamic acid glyceryl ester | 1.0 |
| Silicone-coated titanium oxide fine particles (40 nm) | 4.0 |
| Dimethyl distearyl ammonium hectorite | 0.5 |
| Spherical powder of polyethylene | 3.0 |
| Sodium acrylate/2-acrylamide-2-methylpropane sulfonic acid copolymer (tradename: SIMULGEL EG, SEPPIC) | 0.1 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Mica titanium | 0.1 |
| Phenoxy ethanol | q.s. |
| Purified water | Balance |
| Perfume | q.s. |

| [Example 13] Skin lotion | |
|---|---|
| Ethyl alcohol | 5.0 mass% |
| Glycerin | 1.0 |
| 1,3-Butylene glycol | 5.0 |
| Polyoxyethylene polyoxypropylene decyltetradecyl ether | 0.2 |
| Sodium hexametaphosphate | 0.03 |
| Trimethylglycine | 1.0 |
| Sodium polyaspartate | 0.1 |
| Potassium salt of α-tocopherol 2-L-ascorbic acid phosphoric acid diester | 0.1 |
| Thiotaurine | 0.1 |
| Green tea extract | 0.1 |
| Peppermint extract | 0.1 |
| α-Glucosylhesperidin | 0.01 |
| Magnesium salt of ascorbic acid phosphate | 0.01 |
| Potassium 4-methoxysalicylate | 1.0 |
| Iris root extract | 1.0 |
| Biodynes EMPP | 0.01 |
| Trisodium HEDTA | 0.1 |
| Carboxyvinyl polymer | 0.05 |
| Sodium acrylate/2-acrylamide-2-methylpropane sulfonic acid copolymer (tradename: SIMULGEL EG, SEPPIC) | 0.05 |
| Potassium hydroxide | 0.02 |
| Phenoxy ehtanol | q.s. |
| Purified water | Balance |
| Perfume | q.s. |

| [Example 14] Skin lotion | |
|---|---|
| Ethanol | 10.0 mass% |
| Glycerin | 2.0 |
| Dipropylene glycol | 1.0 |
| Isostearic acid | 0.1 |
| Poly(oxyethylene/oxypropylene)/methylpolysiloxane copolymer | 1.0 |
| Betaine lauryldimethylaminoacetate | 0.1 |
| Citric acid | 0.02 |
| Sodium citrate | 0.08 |
| Sodium hexametaphosphate | 0.01 |
| Hypotaurine | 0.1 |
| Chamomile extract | 0.1 |
| Scutellaria root extract | 0.1 |
| Biodynes EMPP | 1.0 |
| Lavender oil | 0.001 |
| (Ammonium acryloyldimethyltaurate/VP) copolymer (tradename: Aristoflax AVC, Clariant) | 0.01 |
| Phenoxy ethanol | q.s. |
| Activated hydrogen water | 1.0 |
| Purified water | Balance |

| [Example 15] Pack | |
|---|---|
| Ethanol | 10.0 mass% |
| 1,3-Butylene glycol | 6.0 |
| Polyethylene glycol 4000 | 2.0 |
| Olive oil | 1.0 |
| Macadamia nut oil | 1.0 |
| Phytosteryl hydroxystearate | 0.05 |
| Lactic acid | 0.05 |
| Sodium lactate | 0.1 |
| Disodium L-ascorbyl sulfate | 0.1 |
| Potassium salt of α-tocopherol 2-L-ascorbic acid phosphoric acid diester | 0.1 |
| Tocopherol acetate | 0.1 |
| Fish collagen | 0.1 |
| Molasses extract | 1.0 |
| Sodium chondroitin sulfate | 0.1 |
| Biodynes EMPP | 3.0 |
| Sodium carboxymethylcellulose | 0.2 |
| Polyvinyl alcohol | 12.0 |
| (Ammonium acryloyldimethyltaurate/VP) copolymer (tradename: Aristoflex AVC, Clariant) | 2.0 |
| Sodium acrylate/2-acrylamide-2-methylpropane sulfonic acid copolymer (tradename: SIMULGEL EG, SEPPIC) | 0.1 |
| Dow Corning 9041 silicone elastomer blend (blend of dimethicone crosspolymer with dimethylpolysiloxane, Dow Corning) | 3.0 |
| p-Oxybenzoic ester | q.s. |
| Purified water | Balance |
| Perfume | q.s. |

| [Example 16] Powdery solid foundation | |
|---|---|
| Dimethylpolysiloxane | 5.0 mass% |
| Dow Corning 9045 silicone elastomer blend (blend of dimethicone crosspolymer with decamethylcyclopentasiloxane, Dow Corning) | 5.0 |
| Isostearic acid | 0.5 |
| Diisostearyl malate | 3.0 |
| Tri-2-ethylhexanoic acid glyceryl ester | 1.0 |
| Sorbitan sesquiisostearate | 1.0 |
| Spherical PMMA-coated mica | 6.0 |
| Prism-tone powder YR | 1.0 |
| Microgranular zinc oxide | 0.5 |
| Microgranular titanium oxide | 2.0 |
| Synthetic phlogopite | 2.0 |
| Metallic soap-treated talc | 8.0 |
| Spherical silicic anhydride | 5.0 |
| Tocopherol acetate | 0.1 |
| δ-Tocopherol | 0.1 |
| Rosa roxburghii extract | 0.1 |
| Biodynes EMPP | 0.1 |
| p-Oxybenzoic ester | q.s. |
| Methylbis(trimethylsiloxy)silylisopentyl trimethoxycinnamate | 1.0 |
| 2-Ethylhexyl p-methoxycinnamate | 3.0 |
| Spherical polyalkyl acrylate powder | 6.0 |
| Methylhydrogen polysiloxane-coated talc | Balance |
| Methylhydrogen polysiloxane-coated sericite | 5.0 |
| Methylhydrogen polysiloxane-coated titanium oxide | 15.0 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 10.0 |
| Mica titanium | 1.0 |
| Methylhydrogen polysiloxane-coated pigment (coloring material) | 5.0 |

| [Example 17] Powdery solid foundation | |
|---|---|
| Ceresin | 0.5 mass% |
| Dimethylpolysiloxane | 2.0 |
| Methylphenyl polysiloxane (Dimethicone/vinyldimethicone) | 1.0 |
| crosspolymer (tradename: KSG-16, The Shin-Etsu Chemical Co., Ltd.) | 10.0 |
| Squalane | 7.0 |
| Squalane (vegetable) | 1.0 |
| Sorbitan sesquiisostearate | 1.0 |
| Glycerol-modified silicone resin-coated Calcined sericite | 16.0 |
| Glycerol-modified silicone resin-coated sericite | 7.0 |
| Yellow iron oxide-coated mica titanium | 0.1 |
| Microgranular titanium oxide | 5.0 |
| Talc | 10.0 |
| Titanium oxide-coated sericite | 0.1 |
| Boron nitride | 2.5 |
| Red iron oxide-coated mica titanium | 0.1 |
| Phytosterol | 0.1 |
| Ascorbyl dipalmitate | 0.1 |
| DL-α-Tocopherol acetate | 0.1 |
| D-δ-Tocopherol | 0.1 |
| Biodynes EMPP | 0.1 |
| p-Oxybenzoic ester | q.s. |
| 2-Ethylhexyl p-methoxycinnamate | 1.0 |
| Spherical polyalkyl acrylate powder | 8.0 |
| Methylhydrogen polysiloxane-coated mica | Balance |
| Methylhydrogen polysiloxane-coated iron oxide titanium oxide sinter | 5.0 |
| Methylhydrogen polysiloxane-coated sericite | 5.0 |
| Methylhydrogen polysiloxane-coated titanium oxide | 4.0 |
| Methylhydrogen polysiloxane-coated flaky titanium oxide | 5.0 |
| Methylhydrogen polysiloxane-coated pigment (coloring material) | 5.0 |
| Perfume | q.s. |

| [Example 18] Powdery solid foundation | |
|---|---|
| α-Olefin oligomer | 3.0 mass% |
| Vaseline | 3.0 |
| Dow Corning 9040 silicone elastomer blend (blend of dimethicone crosspolymer with decamethylcyclopentasiloxane, Dow Corning) | 3.0 |
| Macadamia nut oil | 0.1 |
| sorbitan sesquiisostearate | 1.0 |
| Alkyl-modified silicone resin-coated yellow iron oxide | 2.0 |
| Alkyl-modified silicone resin-coated red iron oxide | 1.0 |
| Alkyl-modified silicone resin-coated black iron oxide | 0.5 |
| Yellow iron oxide-coated mica titanium | 5.0 |
| Synthetic phlogopite | 5.0 |
| Titanium oxide | 1.0 |
| Zinc oxide | 1.0 |
| Low-temperature fired zinc oxide | 4.0 |
| Calcined sericite | 10.0 |
| Phlogopite | 1.0 |
| Aluminum oxide | 1.0 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 15.0 |
| Talc | Balance |
| Synthetic phlogopite | 5.0 |
| Crosslinked silicone powder (TREFIL E-506) | 10.0 |
| DL-α-Tocopherol acetate | 0.1 |
| D-δ-Tocopherol | 0.1 |
| Biodynes EMPP | 5.0 |
| p-Oxybenzoic ester | q.s. |
| 2-Ethylhexyl p-methoxycinnamate | 1.0 |
| Calcium alginate powder | 1.0 |
| Perfume | q.s. |

| [Example 19] Solid foundation | |
|---|---|
| α-Olefin oligomer | 10.0 mass% |
| Microcrystalline wax | 0.5 |
| Dow Corning 9041 silicone elastomer blend (blend of dimethicone crosspolymer with dimethylpolysiloxane, Dow Corning) | 3.0 |
| Ceresin | 5.0 |
| Dimethylpolysiloxane | 15.0 |
| Methylphenyl polysiloxane | 10.0 |
| Macademia nut oil | 0.1 |
| Carnauba wax | 0.1 |
| Tri-2-ethylhexanoic acid glyceryl ester | 7.0 |
| Cetyl 2-ethylhexanoate | 10.0 |
| Sorbitan sesquiisostearate | 1.5 |
| Mica | 0.5 |
| Aluminum stearate | 1.0 |
| Crosslinked silicone powder (TREFIL E-506) | 8.0 |
| N-Lauroyl-L-lysine | 0.1 |
| D-δ-Tocopherol | q.s. |
| Biodynes EMPP | 0.1 |
| Red iron oxide | q.s. |
| Yellow iron oxide | q.s. |
| Calcium alginate powder | 1.0 |
| Nylon powder | Balance |
| Spherical silicic anhydride | 1.0 |
| Titanium oxide | 1.0 |

| [Example 20] Emulsified foundation | |
|---|---|
| Microcrystalline wax | 1.0 mass% |
| Dimetyl polysiloxane | 15.0 |
| Decamethylcyclopentasiloxane | 2.0 |
| Dow Corning 9045 silicone elastomer blend (blend of dimethicone crosspolymer with decamethylcyclopentasiloxane, Dow Corning) | 3.0 |
| 3-Tris(trimethylsiloxy)silylpropylcarbamide acid pullulan | 0.5 |
| 1,3-Butylene glycol | 6.0 |
| Candelilla wax | 3.0 |
| Isostearic acid | 1.0 |
| Ethylene glycol fatty acid ester | 0.1 |
| Lanolin fatty acid octyldodecyl ester | 0.5 |
| 2-Alkyl-N-carboxymethyl-N-hydroxyethylimidazolinium betaine | 4.0 |
| Titanium oxide | 7.5 |
| Barium sulfate | 5.0 |
| Talc | 3.0 |
| Silicic anhydride | 4.0 |
| Crosslinked silicone powder (TREFIL E-506) | 0.1 |
| Sodium metaphosphate | 0.1 |
| Hydroxypropyl-β-cyclodextrin | 0.1 |
| DL-α-Tocopherol acetate | 0.1 |
| Hamamelis extract | 0.1 |
| Paeonia lactiflora extract | 0.1 |
| Sodium chondroitin sulfate | 0.1 |
| Sodium hyaluronate | 0.1 |
| Biodynes EMPP | 0.1 |
| Gambir extract | 0.1 |
| p-Oxybenzoic ester | q.s. |
| Red iron oxide | q.s. |
| Yellow iron oxide | q.s. |
| Black iron oxide | q.s. |
| Flaky inorganic composite powder (talc which has been coated with titanium oxide, aluminum oxide, and silicic anhydride, tradename: COVERLEAF AR-80, Catalysts and Chemicals Industries Co., Ltd.) | 1.0 |
| Xanthan gum | 0.1 |
| Sodium carboxymethylcellulose | 0.1 |
| Sodium acrylate/2-acrylamide-2- methylpropane sulfonic acid copolymer (tradename: SIMULGEL EG, SEPPIC) | 0.5 |
| Melilot extract | 2.0 |
| Purified water | Balance |

| [Example 21] W/O foundation | |
|---|---|
| Dimethyl polysiloxane | 3.0 mass% |
| Decamethylcyclopentasiloxane | 10.0 |
| Polyoxyethylene/methylpolysiloxane copolymer | 3.0 |
| Dodecamethylcyclohexasiloxane | 5.0 |
| 3-Tris(trimethylsiloxy)silylpropylcarbamide acid pullulan | 0.1 |
| (Dimethicone/vinyldimethicone) crosspolymer (tradename: KSG-16, The Shin-Etsu Chemical Co., Ltd.) | 5.0 |
| Glycerin | 4.0 |
| 1,3-Butylene glycol | 5.0 |
| Palmitic acid | 0.5 |
| Distearyldimethyl ammonium chloride | 0.2 |
| Metallic soap-treated talc | 2.0 |
| Crosslinked silicone powder (TREFIL E-506) | 0.1 |
| Red iron oxide-coated mica titanium | 0.5 |
| N-Lauroyl-L-lysine | 2.0 |
| Sodium L-glutamate | 2.0 |
| Tocopherol acetate | 0.1 |
| δ-Tocopherol | 0.1 |
| p-Oxybenzoic ester | q.s. |
| Phenoxy ethanol | 0.2 |
| Spherical nylon powder | 1.0 |
| Spherical polyalkyl acrylate powder | 3.0 |
| Melilot extract | 2.0 |
| Blackberry lily extract | 1.0 |
| Biodynes EMPP | 5.0 |
| Purified water | Balance |
| Dextrin fatty acid-treated talc | 3.0 |
| Dextrin fatty acid-treated titanium dioxide | 15.0 |
| Dextrin fatty acid-treated yellow iron oxide | 3.0 |
| Dextrin fatty acid-treated black iron oxide | 0.5 |

| [Example 22] O/W foundation | |
|---|---|
| Dimethyl polysiloxane | 8.0 mass% |
| Dow Corning 9040 silicone elastomer blend (blend of dimethicone crosspolymer with decamethylcyclopentasiloxane, Dow Corning) | 20.0 |
| Behenyl alcohol | 0.5 |
| Batyl alcohol | 0.5 |
| 1,3-Butylene glycol | 5.0 |
| Macadamia nut oil | 0.1 |
| Isostearic acid | 1.5 |
| Stearic acid | 1.0 |
| Behenic acid | 0.5 |
| Cetyl 2-ethylhexanoate | 5.0 |
| Polyoxyethylene glyceryl monostearate | 1.0 |
| Self-emulsifiable glyceryl monostearate | 1.0 |
| Yellow iron oxide-coated mica titanium | 2.0 |
| Titanium oxide | 4.0 |
| Talc | 0.5 |
| Kaolin | 3.0 |
| Synthetic phlogopite | 0.1 |
| Crosslinked silicone powder | 0.1 |
| Silicic anhydride | 5.0 |
| Potassium hydroxide | 0.2 |
| Triethanolamine | 0.8 |
| DL-α-Tocopherol acetate | 0.1 |
| Sodium hyaluronate | 0.1 |
| Biodynes EMPP | 0.1 |
| Thymus serpyllum extract | 0.1 |
| p-Oxybenzoic ester | q.s. |
| 2-Ethylhexyl p-methoxycinnamate | 1.0 |
| Red iron oxide | q.s. |
| Yellow iron oxide | q.s. |
| Black iron oxide | q.s. |
| Xanthan gum | 0.1 |
| Sodium acrylate/2-acrylamide-2-methylpropane sulfonic acid copolymer (tradename: SIMULGEL EG, SEPPIC) | 1.0 |
| Bentonite | 1.0 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 5.0 |
| Sodium carboxymethylcellulose | 0.1 |
| Purified water | Balance |
| Perfume | q.s. |

| [Example 23] Foundation | |
|---|---|
| Dodecamethylcyclohexasiloxane | 15.0 mass% |
| Decamethylcyclopentasiloxane | Balance |
| 3-Tris(trimethylsiloxy)silylpropylcarbamide acid pullulan | 5.0 |
| Dow Corning 9041 silicone elastomer blend (blend of dimethicone crosspolymer with dimethylpolysiloxane, Dow Corning) | 30.0 |
| Ethanol | 10.0 |
| Isostearic acid | 0.5 |
| Myristic acid-treated zinc oxide | 0.5 |
| Dextrin palmitate-coated titanium oxide | 10.0 |
| Dextrin palmitate-coated talc | 7.0 |
| Titanium oxide with silicone-treated surface (30 nm) | 5.0 |
| Crosslinked silicone powder | 1.0 |
| Spherical silicic anhydride | 2.0 |
| L-ascorbyl magnesium phosphate | 0.2 |
| DL-α-Tocopherol acetate | 0.1 |
| D-δ-Tocopherol | 0.1 |
| Glutathione | 0.1 |
| Sophora angustifolia extract | 0.1 |
| Bupleurum extract | 1.0 |
| Biodynes EMPP | 2.0 |
| 2-Ethylhexyl p-methoxycinnamate | 5.0 |
| Dextrin palmitate-coated red iron oxide | q.s. |
| Dextrin palmitate-coated yellow iron oxide | q.s. |
| Dextrin palmitate-coated black iron oxide | q.s. |
| Perfume | q.s. |

| [Example 24] Makeup base | |
|---|---|
| Dimethyl polysiloxane | 5.0 mass% |
| 3-Tris(trimethylsiloxy)silylpropylcarbamide acid pullulan | 0.1 |
| Dow Corning 9045 silicone elastomer blend (blend of dimethicone crosspolymer with decamethylcyclopentasiloxane, Dow Corning) | 30.0 |
| Decamethylcyclopentasiloxane | Balance |
| Ethyl alcohol | 8.0 |
| Iron blue-coated mica titanium | 0.5 |
| Flaky inorganic composite powder (talc which has been coated with titanium oxide, aluminum oxide, and silicic anhydride, tradename: COVERLEAF AR-80, Catalysts and Chemicals Industries Co., Ltd.) | 5.0 |
| Methylsiloxane network polymer | 5.0 |
| Crosslinked silicone powder (TREFIL E-506) | 5.0 |
| Citric acid | 0.02 |
| Sodium citrate | 0.08 |
| Tocopherol acetate | 0.1 |
| δ-Tocopherol | 0.1 |
| Melilot extract | 3.0 |
| Turmeric extract | 0.01 |
| Biodynes EMPP | 0.1 |
| Purified water | 1.0 |
| Poly(oxyethylene/oxypropylene)/methylpolysiloxane copolymer | 5.0 |

| [Example 25] Makeup base | |
|---|---|
| Dimethyl polysiloxane | 5.0 mass% |
| 3-Tris(trimethylsiloxy)silylpropylcarbamide acid pullulan | 1.0 |
| (Dimethicone/vinyldimethicone) crosspolymer (tradename: KSG-16, The Shin-Etsu Chemical Co., Ltd.) | 15.0 |
| Decamethylcyclopentasiloxane | 25.0 |
| Polyoxyethylene/methylpolysiloxane copolymer | 3.0 |
| Glycerin | 1.0 |
| 1,3-Butylene glycol | 5.0 |
| Xylite | 0.5 |
| Isostearic acid | 0.5 |
| Alkyl-modified silicone resin-coated silicic anhydride | 2.0 |
| Talc | 0.5 |
| Aluminum stearate | 1.0 |
| Red iron oxide-coated mica titanium | 0.1 |
| Sodium hexametaphosphate | 0.05 |
| Dipotassium glycyrrhizinate | 0.1 |
| L-Serine | 0.1 |
| Hypericum erectum extract | 0.1 |
| DL-α-Tocopherol acetate | 0.2 |
| Thiotaurine | 0.1 |
| Rosa roxburghii Tratt extract | 0.1 |
| Paeonia lactiflora extract | 0.1 |
| Lysolecithin | 0.01 |
| Acetylated sodium hyaluronate | 0.1 |
| Saxifraga stolonifera extract | 0.1 |
| Biodynes EMPP | 0.1 |
| p-Oxybenzoic ester | q.s. |
| Phenoxy ethanol | q.s. |
| Dextrin palmitate-coated yellow iron oxide | 0.1 |
| Dimethyl distearyl ammonium hectorite | 1.0 |
| Purified water | Balance |
| Trimethylsiloxysilicic acid | 1.5 |
| Spherical silicic anhydride | 1.0 |
| Spherical polyethylene powder | 5.0 |
| Perfume | q.s. |

### INDUSTRIAL APPLICABILITY

The anti-aging compositions, collagen production promoting compositions and the like according to the present invention have excellent collagen production promotion effect, and are safe. Therefore, the collagen production promoting composition and the collagen production promotion methods according to the present invention can promote collagen production to maintain the collagen level and, in addition, can promote collagen gel contraction and integrin production in fibroblast or epidermal cell to effectively prevent or ameliorate wrinkle and sagging of the skin.

## Claims

1. An anti-aging composition **characterized by** comprising a collagen production promoting composition comprising, as an active ingredient, a hydrogen peroxide-treated yeast hydrolysate which has been cultured in a nutrient medium containing a non-animal-derived glycosaminoglycan.

2. A collagen production promoting composition **characterized by** comprising, as an active ingredient, a hydrogen peroxide-treated yeast hydrolysate which has been cultured in a nutrient medium containing a non-animal-derived glycosaminoglycan.

3. A method for promoting collagen production, **characterized by** comprising using a hydrogen peroxide-treated yeast hydrolysate, which has been cultured in a nutrient medium containing a non-animal-derived glycosaminoglycan, to promote the production of collagen.

4. An anti-aging composition **characterized by** comprising a collagen gel contraction promoting composition comprising, as an active ingredient, a hydrogen peroxide-treated yeast hydrolysate which has been cultured in a nutrient medium containing a non-animal-derived glycosaminoglycan.

5. A collagen gel contraction promoting composition **characterized by** comprising, as an active ingredient, a hydrogen peroxide-treated yeast hydrolysate which has been cultured in a nutrient medium containing a non-animal-derived glycosaminoglycan.

6. A method for promoting collagen gel contraction, **characterized by** comprising using a hydrogen peroxide-treated yeast hydrolysate, which has been cultured in a nutrient medium containing a non-animal-derived glycosaminoglycan, to promote the contraction of a collagen gel.

7. An anti-aging composition **characterized by** comprising an integrin production promoting composition for promoting fibroblast integrin production, said integrin production promoting composition comprising, as an active ingredient, a hydrogen peroxide-treated yeast hydrolysate which has been cultured in a nutrient medium containing a non-animal-derived glycosaminoglycan.

8. An integrin production promoting composition for promoting fibroblast integrin production, **characterized in that** said integrin production promoting composition comprises, as an active ingredient, a hydrogen peroxide-treated yeast hydrolysate which has been cultured in a nutrient medium containing a non-animal-derived glycosaminoglycan.

9. A method for promoting fibroblast integrin production, **characterized by** comprising using a hydrogen peroxide-treated yeast hydrolysate, which has been cultured in a nutrient medium containing a non-animal-derived glycosaminoglycan, to promote fibroblast integrin production.

10. An anti-aging composition **characterized by** comprising an integrin production promoting composition for promoting epidermal cell integrin production, said integrin production promoting composition comprising, as an active ingredient, a hydrogen peroxide-treated yeast hydrolysate which has been cultured in a nutrient medium containing a non-animal-derived glycosaminoglycan.

11. An integrin production promoting composition for promoting epidermal cell integrin production, **characterized by** comprising, as an active ingredient, a hydrogen peroxide-treated yeast hydrolysate which has been cultured in a nutrient medium containing a non-animal-derived glycosaminoglycan.

12. A method for promoting epidermal cell integrin production, **characterized by** comprising using a hydrogen peroxide-treated yeast hydrolysate, which has been cultured in a nutrient medium containing a non-animal-derived glycosaminoglycan, to promote epidermal cell integrin production.
